**Europäisches Patentamt**

**European Patent Office**

**Office européen des brevets**

(11) Veröffentlichungsnummer: **0 009 621**
**B1**

(12) **EUROPÄISCHE PATENTSCHRIFT**

(45) Veröffentlichungstag der Patentschrift: **15.04.81**

(21) Anmeldenummer: **79103193.3**

(22) Anmeldetag: **29.08.79**

(51) Int. Cl.³: **C 07 C 85/12, C 07 C 87/10**

(54) Verfahren zur Herstellung von Triäthylamin.

(30) Priorität: **08.09.78 DE 2839135**

(43) Veröffentlichungstag der Anmeldung:
**16.04.80 Patentblatt 80/8**

(45) Bekanntmachung des Hinweises auf die Patenterteilung:
**15.04.81 Patentblatt 81/15**

(84) Benannte Vertragsstaaten:
**DE FR GB IT NL**

(56) Entgegenhaltungen:
**DE - A - 1 768 853**
**DE - A - 2 519 838**
**US - A - 3 264 354**
**US - A - 3 673 251**

(73) Patentinhaber: **EC ERDÖLCHEMIE GMBH**
**Postfach 75 2002**
**D-5000 Köln 71 (DE)**

(72) Erfinder: **Mainusch, Klaus, Dr.**
**Gneisenaustrasse 15a**
**D-4047 Dormagen 1 (DE)**
Erfinder: **Schleppinghoff, Bernhard, Dr.**
**Adolf-Kolping-Strasse 5**
**D-4047 Dormagen 1 (DE)**
Erfinder: **Munter, Klaus**
**Herrenweg 51**
**D-4047 Dormagen 5 (DE)**

(74) Vertreter: **Dill, Erwin, Dr. et al,**
**c/o BAYER AG Zentralbereich Patente Marken**
**und Lizenzen Bayerwerk**
**D-5090 Leverkusen 1 (DE)**

Courier Press, Leamington Spa, England.

## Verfahren zur Herstellung von Triäthylamin

Die vorliegende Erfindung betrifft ein Verfahren zur kontinuierlichen Herstellung von Triäthylamin durch katalytische Hydrierung von Acetonitril in der Gasphase.

Bei der Acrylnitrilproduktion nach dem Sohio-Verfahren (Ullmanns Encyclopädie der technischen Chemie, Band 7, 4. Auflage, Seite 96, Verlag Chemie, Weinheim/Bergstraße 1974) fallen etwa 40 kg Acetonitril pro 1 Tonne Acrylnitril als Koppelprodukt an, das wirtschaftlich genutzt werden muß. Eine der Weiterverarbeitungsmöglichkeiten besteht darin, Acetonitril in Triäthylamin zu überführen.

Gasphasenhydrierungen des Acetonitrils sind bereits bekannt. So wird z.B. in der GB—PS 1 239 505 die Hydrierung von Acetonitril bei einem Wasserstoffdruck von 36 bis 42 bar und einer Temperatur von 150°C (Beispiele 1 bis 3) an einem Chrom/Nickel-Katalysator beschrieben, wobei 2,3 bis 88% Monoäthylamin gebildet werden. Bei 5 bar/150°C und 230 bar/150°C (Beispiele 4 und 5) werden 68 bzw. 28% Monoäthylamin neben Di- und Triäthylamin erhalten. Nachteilig bei diesem Verfahren sind die geringe Raumgeschwindigkeit (Liquid hourly space velocity "LHSV") von nur 0,33 Volumen Acetonitril pro Volumen Katalysator und Stunde "v/v.h" (600 ml/h Acetonitril pro 2 Liter Katalysator gemäß Beispiel 1—5) sowie der Zwangsanfall des minderwertigen Monoäthylamins und des Diäthylamins. Beispielsweise wird bei 100%igem Umsatz ein Äthylamingemisch folgender Zusammensetzung erhalten: Monoäthylamin 2,3 Gew.-%; Diäthylamin 8,4 Gew.-%; Triäthylamin 89,3 Gew.-%.

Von entscheidendem Nachteil bei diesem Verfahren sind die relativ hohen Betriebsdrücke von 42 bzw. 50 bar sowie Temperaturen von 150°C, die jeweils selektivitätsvermindernde Nebenreaktionen begünstigen. Auch erfordert die Anwendung derartig hoher Drücke erhöhte Investionsausgaben, was sich als kostenmäßiger Nachteil auswirkt.

Ein weiterer wesentlicher Nachteil ist darin zu sehen, daß das Hauptprodukt Triäthylamin mit ungenügender Selektivität gebildet wird. Im rohen Hydrierprodukt sind die beiden Nebenprodukte Di- und Monoäthylamin mit 10,7 Gew.-% vorhanden. Dadurch wird die Reingewinnung des Triäthylamins technisch sehr aufwendig und verteuert sich erheblich.

In der vorgenannten Patentschrift werden auch keine Angaben über die Katalysatorstandzeiten gemacht. In der Tat hat sich das dort vorgeschlagene Verfahren für eine technische Anwendung zur wirtschaftlichen Herstellung von Triäthylamin als ungeeignet erwiesen.

Es wurde nun gefunden, daß die oben aufgeführten Nachteile bei der kontinuierlichen Herstellung von Triäthylamin durch katalytische Hydrierung von Acetonitril in der Gasphase bei Temperaturen von 80°C bis 115°C und Drücken von 1 bis 60 bar vermieden werden können, wenn man die Hydrierung in Gegenwart eines Edelmetalls der VIII. Gruppe des Periodensystems (Mendelejew) auf Lithium-Aluminium-Spinell als Träger, gegebenenfalls in Gegenwart von bis zu etwa 5 Gew.-% Mono- und Diethylamin, bezogen auf Acetonitril, durchführt.

Der Trägerkatalysator kann in jeder beliebigen Form (Kugeln, Zylinder, Extrudate, Hohlstränge, Granulate oder Schuppen) in einer Größe von 1 bis 8 mm, bevorzugt 3 bis 6 mm, Durchmesser eingesetzt werden. Die bevorzugte Form ist die Kugelform.

Der Lithium-Aluminium-Spinell-Träger wird bevorzugt aus Aluminiumoxid mit einer spezifischen Oberfläche von 20 bis 400 m²/g hergestellt.

Der erfindungsgemäße Lithium-Aluminium-Katalysatorträger ist an sich bekannt. Seine Herstellung wird z.B. in der DT—PS 1 249 255 beschrieben.

Als Edelmetallkatalysatoren der VIII. Hauptgruppe seien genannt Rhodium, Palladium, Iridium oder Platin. Die genannten Metalle können allein oder als Gemisch miteinander eingesetzt werden. Man kann ihnen auch Vanadin und/oder Kupfer als Promotor zusetzen. Bevorzugt werden Palladium, Platin- oder Palladium/Platin-Katalysatoren mit und ohne Vanadin-Zusätze verwendet. Besonders bevorzugt sind Palladium/Vanadin-, Platin/Vanadin- sowie Palladium/Platin/Vanadin-Katalysatoren für das erfindungsgemäße Verfahren geeignet. Im allgemeinen enthält der Trägerkatalysator 0,1 bis 5,0 Gew.-%, vorzugsweise 0,5 bis 2,0 Gew.-% des Edelmetalls der VIII. Gruppe des Periodensystems.

Falls zusätzlich Vanadin als Promotor-Komponente im Katalysator enthalten sein soll, so beträgt der Vanadin-Gehalt im fertigen Katalysator 0,1 bis 2,0 Gew.-%, bevorzugt 0,2 bis 0,8 Gew.-%.

Die für die Durchführung des erfindungsgemäßen Verfahrens verwendeten Katalysatoren sind an sich bekannt. Ihre Herstellung wird z.B. in der DT—OS 2 519 838 beschrieben.

Das Edelmetall, z.B. Palladium oder Platin, wird dabei auf den Träger in bekannter Weise aufgebracht. Beispielsweise wird das Edelmetall, z.B. Platin, in Mengen von 0,1 bis 5 Gew.-%, vorzugsweise 0,5 bis 2 Gew.-%, bezogen auf den fertigen Katalysator, auf den Träger aufgebracht. Zu diesem Zweck tränkt oder besprüht man den Träger beispielsweise mit einer wäßrigen Edelmetallsalz- z.B. Platinsalzlösung. Für die Tränkung oder Besprühung sind alle handelsüblichen Edelmetallverbindungen geeignet. Vor der Reduktion der Edelmetallverbindungen in die metallische Form können die Edelmetallverbindungen zu Edelmetallhydroxid oder Edelmetalloxid umgewandelt werden. Die normalerweise nachfolgende Reduktion der Edelmetallverbindungen zum Metall kann beispielsweise mit Formaldehyd und Hydrazin in neutraler oder alkalischer Lösung oder mit Ameisensäure, Wasserstoff, Kohlenoxid oder Äthylen durchgeführt werden. Es können jedoch auch andere Reduktionsmethoden angewendet werden. Falls zusätzlich Vanadin als aktive Komponente im

**0 009 621**

Katalysator enthalten sein soll, so kann das Vanadin vor oder nach dem Aufbringen der Hauptkomponente, z.B. das Edelmetalls, in Form von löslichen Vanadiumverbindungen, z.B. Vanadyloxalat, auf den Träger aufgebracht werden. Im allgemeinen wird sodann das auf den Träger aufgebrachte Vanadiumsalz durch Tempern bei Temperaturen von etwa 200 bis etwa 500°C in die Oxidform übergeführt. Der Gehalt an Vanadin im fertigen Katalysator kann 0,1 bis 2 Gew.-%, bevorzugt 0,2 bis 0,8 Gew.-%, betragen. Es ist zweckmäßig, vor Einsatz der so hergestellten Katalysatoren die von der Herstellung her enthaltenen Anionen mit destilliertem Wasser auszuwaschen.

Die genannten erfindungsgemäßen Katalysatoren erwiesen sich als ausgezeichnete Hydrierkontakte, die auch nach mehr als 500 betriebsstunden unverändert aktiv und selektiv waren.

Die Durchführung des erfindungsgemäßen Verfahrens kann im allgemeinen in der Weise erfolgen, daß man in einem Reaktor Wasserstoff und Acetonitril sowie das Monoäthylamin gasförmig durch den im Festbett angeordneten Katalysator leitet. Bei einem derartigen Hydrierreaktor kann es sich z.B. um einen Reaktor mit einem oder mehreren Reaktionsrohren handeln, die mit einem Kühlmittel umspült werden.

Für das erfindungsgemäße Verfahren eignet sich ein Druckbereich von 1 bar bis 60 bar, bevorzugt 1 bar bis 50 bar, besonders bevorzugt 5 bis 35 bar. Die Reaktionstemperatur beträgt erfindungsgemäß etwa 80°C bis 115°C, bevorzugt etwa 85 bis 110°C.

Bei dem erfindungsgemäßen Verfahren werden im allgemeinen Mengen von etwa 460 bis 620 ml/h Acetonitril pro 1 Katalysator durchgesetzt. Das entspricht einer Raumgeschwindigkeit von etwa 0,5 bis 0,6 Volumen Acetonitril pro Volumen Katalysator und Stunde.

Als Einsatzprodukt in die Hydrierung eignet sich ein Acetonitril jeglicher Herkunft mit einer Reinheit von 90 Gew.-% bis 99,9 Gew.-%. Vorzugsweise wird Acetonitril eingesetzt, das als Nebenprodukt bei der Acrylnitril-Herstellung anfällt und eine Reinheit von etwa 95 Gew.-% bis 99,8 Gew.-% aufweist.

Die erfindungsgemäße Hydrierung des Acetonitrils kann in Gegenwart von 0 bis 5,0, bevorzugt 0,1 bis 3,0 Gew.-% Mono- und Diäthylamin, bezogen auf Acetonitril, durchgeführt werden. Im allgemeinen verfährt man dabei so, daß man Acetonitril mit dem vorgenannten Reinheitsgrad in die Hydrierung einsetzt und das nach dem ersten Durchgang in einer Menge von bis 5, bevorzugt 0,1 bis 3,0 Gew.-% im Reaktionsgemisch enthaltene Mono- und Diäthylamin destillativ abtrennt und in die Hydrierung zurückführt. Diese Mono- und Diäthylamin-Abtrennung und -Rückführung kann nach Anfahren der Hydrierung kontinuierlich erfolgen.

Die Aufarbeitung des Hydrierproduktes kann kontinuierlich oder diskontinuierlich durchgeführt werden unter Anwendung von an sich bekannten Methoden. Bevorzugt ist eine kontinuierliche Trennung des Hydriergemisches unter Isolierung von reinem Triäthylamin. Dazu kann beispielsweise in einer nachgeschalteten Kolonne nach Druckentspannung der im Reaktionsgemisch enthaltene Ammoniak abgestrippt werden oder in einer Druckdestillation als Kopfprodukt abgenommen und einer weiteren Verwendung zugeführt werden. Das flüssige Sumpfprodukt wird einer zweiten Destillation zugeführt, bei der im Sumpf oder einige Böden darüber reines Triäthylamin gewonnen wird. Das Kopfprodukt dieser Fraktionierungsstufe besteht im wesentlichen aus Diäthylamin und gegebenenfalls nicht umgesetztem Acetonitril und kann wieder in die Hydrierung zurückgeführt werden.

Zusammenfassend läßt das erfindungsgemäße Verfahren folgende entscheidende Vorteile erkennen:

1. Durch Verwendung von sehr aktiven und selektiven Katalysatoren kann die Hydrierung von Acetonitril bei überraschend niedrigen Temperaturen und Drücken durchgeführt werden. Dieser Umstand ermöglicht minimale Herstellungskosten.

2. Infolge der außergewöhnlich hohen Selektivität der erfindungsgemäßen Katalysatoren wird ein Reaktionsprodukt erhalten, das überwiegend aus Triäthylamin besteht und praktisch frei von Monoäthylamin ist. Dadurch wird eine äußerst einfache und billige Reingewinnung von Triäthylamin ermöglicht.

3. Die im erfindungsgemäßen Verfahren zu verwendenden Katalysatoren zeichnen sich durch hohe Standzeiten aus.

4. Die Wirtschaftlichkeit des Acrylnitril-Verfahrens wird durch die Überführung des Koppelproduktes Acetonitril zu Triäthylamin erhöht.

Die nachfolgenden Beispiele sollen die erfindungsgemäße Arbeitsweise näher erläutern.

## Beispiele 1 bis 6

Die Versuche wurden in der Gasphase in einem V2A-Rohrreaktor von 24 mm Durchmesser und 1,05 m Länge durchgeführt. Das Reaktionsrohr, das mit ca. 325 ml eines Katalysators gefüllt war, war mit einem Mantelrohr umgeben, das mit Heiz- oder Kühlmedium durchströmt wurde. Die verwendeten Katalysatoren bestanden aus Lithium-Aluminium-Spinell als Trägermaterial und wurden gemäß DT—OS 2 519 838 hergestellt. Die Metalldotierungen und spezifischen Oberflächen sind in Tabelle 1 angegeben. Durch das Reaktionsrohr wurde von oben her 100 ml/h 99,0%iges Acetonitril, welches ein aufgearbeitetes Nebenprodukt der Acrylnitrilherstellung war, und ca. 160 Nl/h Wasserstoff gepumpt.

3

# 0 009 621

Die mittlere Reaktionstemperatur betrug ca. 105°C; der Druck betrug 26 Atmosphären. Das Reaktionsgemisch wurde bei Normaltemperatur kondensiert. Aus der Gasphase wurde ein gewisser Anteil als Abgas entspannt. Das vom Wasserstoff und Ammoniak befreite Hydriergemisch wurde analysiert. Die erzielten Ergebnisse sind in Tabelle 1 zusammengefaßt.

| Beispiel | Katalysator aktives Metall in Gew.-% | Oberfl. $m^2/g$ | gebildetes Produkt in Gew.-% bezogen auf die Gesamtamine | | |
|---|---|---|---|---|---|
| | | | Mono- | Di- | Triethylamin |
| 1 | 1,8% Pd | 38 | <0,1 | 0,5 | 99,5 |
| 2 | 1,8% Pt | 120 | <0,1 | 0,5 | 99,5 |
| 3 | 1,8% Pd/0,6% V | 23 | <0,1 | 0,4 | 99,6 |
| 4 | 1,8% Pt/0,6% V | 340 | 0,1 | 0,3 | 99,6 |
| 5 | 0,9% Pd/0,9 Pt | 290 | <0,1 | 0,5 | 99,5 |
| 6 | 0,9% Pd/0,9% Pt 0,6% V | 364 | <0,1 | 0,3 | 99,7 |

Der Umsatz war vollständig. Das Hydriergemisch enthielt praktisch kein Monoäthylamin und nur Spuren an Diäthylamin. Die Versuche wurden nach jeweils 500 h Laufzeit ohne Katalysatordesaktivierung abgebrochen.

Beispiele 7 bis 9
(zum Vergleich)

Es wurde in der gleichen Weise wie in den Beispielen 1—6 verfahren, jedoch wurden andere als die erfindungsgemäßen Katalysatoren eingesetzt. Die verwendeten Katalysatoren und die damit erhaltenen Ergebnisse sind in den Tabellen 2 und 3 zusammengefaßt.

Tabelle 2
Katalysatoren der Vergleichsbeispiele

| Katalysator | $O(m^2/g)$ | Hersteller | Bezeichnung |
|---|---|---|---|
| A Mo (11%)/Co (4%) auf $Al_2O_3$ | 300 | BASF | M8—10 |
| B Ni (60%) auf $SiO_2$ | 148 | Harshaw | NiO104 T 1/8" |
| C Pt (0,8%) auf $SiO_2/Al_2O_3$ | 184 | Universal Mathey | UM 37 |

Tabelle 3
Vergleichsweise Katalysatoren und Ergebnisse

| Vergleichsbeispiele | gebildetes Produkt in Gew.-% bezogen auf die Gesammtamine | | | Umsatz in Gew.-% | Bemerkungen |
|---|---|---|---|---|---|
| | Mono- | Di- | Triethylamin | | |
| 7 | 37,2 | 48,0 | 14,8 | 16,8 ⎱ | Nach 15 Betriebsstunden wegen Druckantiegs abgestellt (Polymerisate) |
| 8 | 62,5 | 31,2 | 6,3 | 60,3 ⎰ | |
| 9 | 1,2 | 19,4 | 79,4 | 43,0 | Nach 20 Betriebsstunden wegen zu geringen Umsatzes abgestellt |

4

Die Umsätze des Acetonitrils waren deutlich geringer. Nach sehr kurzer Beitriebszeit (siehe Tabelle 3) mußten die Versuche wegen Polymerisation im Reaktor und/oder zunehmender Desaktivierung der Katalysatoren abgebrochen werden.

Beispiel 10
(zum Vergleich)
Es wurde wie im Beispiel 3 verfahren, jedoch der Versuch bei 150°C Reaktionstemperatur durchgeführt. Aus dem Acetonitril wurde bei vollständigem Umsatz ein Äthylamingemisch mit folgenden Anteilen gebildet (Angaben in Gew.-%, bezogen auf die Gesamtamine):

Monoäthylamin: 9,5%

Diäthylamin: 41,0%

Triäthylamin: 49,5%

Beispiel 11
Es wurde wie im Beispiel 3 verfahren, jedoch wurde der Versuch bei einem Druck von 17 Atmosphären durchgeführt. Aus dem Acetonitril wurde bei vollständigem Umsatz ein Äthylamingemisch mit folgenden Anteilen gebildet (Gew.-%, bezogen auf die Gesamtamine):

Monöathylamin: <0,1%

Diäthylamin: 0,4%

Triäthylamin: 99,6%

Auch nach 500 Stunden kontinuierlicher Laufzeit wurden die gleichen Werte für die Triäthylamin-Selektivität erreicht.

Beispiel 12
Es wurde wie im Beispiel 1 verfahren. Das den Reaktor verlassende Reaktionsgemisch wurde kontinuierlich aufgearbeitet. In der ersten Fraktionierungsstufe wurde bei einem Druck von 16 Atmosphären Ammoniak über Kopf abgenommen. Der Sumpfstrom wurde nach Entspannung auf 2,0 Atmosphären Druck einer zweiten Fraktionierung unterzogen. Der abgezogene Sumpfstrom enthielt 99,5%iges Triäthylamin mit einer Gesamtausbeute von 96,5% der Theorie. Der Destillatstrom dieser zweiten Fraktionierung setzt sich im wesentlichen aus Diäthylamin und Spuren von Triäthylamin zusammen und wird in die Hydrierung zurückgeführt. Es wurde im Abscheider ein Äthylamingemisch folgender Zusammensetzung erhalten (Gew.-%, bezogen auf die Gesamtamine).

Monoäthylamin: <0,1%

Diäthylamin: 0,4%

Triäthylamin: 99,6%

Der Umsatz an Acetonitril war quantitativ. Der Versuch wurde nach 500 Betriebsstunden ohne erkennbare Katalysatordesaktivierung abgebrochen.

## Patentansprüche

1. Verfahren zur kontinuierlichen Herstellung von Triethylamin durch katalytische Hydrierung yon Acetonitril in der Gasphase bei Temperaturen von 80 bis 115 und Drücken von 1 bis 60 bar, dadurch gekennzeichnet, daß man die Hydrierung in Gegenwart eines Edelmetalls der VIII. Gruppe des Periodensystems (Mendelejew) auf Lithium-Aluminium-Spinell als Träger, gegebenenfalls in Gegenwart von bis zu etwa 5 Gew.-% Mono- und Diethylamin, bezogen auf Acetonitril, durchführt.

2. Verfahren nach Anspruch 1, dadurch gekennzeichnet, daß man das im Reaktionsgemisch enthaltene Mono- und Diethylamin destillativ abtrennt und in die Hydrierung zurückführt.

3. Verfahren nach Ansprüchen 1 und 2, dadurch gekennzeichnet, daß man das nach dem ersten Durchgang in einer Menge von bis zu etwa 5 Gew.-% im Reaktionsgemisch enthaltene Mono- und Diethylamin destillativ abtrennt und in die Hydrierung zurückführt, wobei sodann die Mono- und Diethylamin-Abtrennung und -Rückführung kontinuierlich vorgenommen werden.

4. Verfahren nach Ansprüchen 1 bis 3, dadurch gekennzeichnet, daß man die Hydrierung an einem Trägerkatalysator durchführt, der 0,1 bis 5 Gew.-% eines Edelmetalls der VIII. Gruppe des Periodensystems enthält.

5. Verfahren nach Ansprüchen 1 bis 4, dadurch gekennzeichnet, daß man die Hydrierung in Gegenwart eines zusätzlich Vanadin enthaltenden Katalysators durchführt.

6. Verfahren nach Ansprüchen 1 bis 5, dadurch gekennzeichnet, daß man die Hydrierung in Gegenwart eines Katalysators durchführt, der zusätzlich 0,1 bis 2,0 Gew.-% Vanadin enthält.

7. Verfahren nach Ansprüchen 1 bis 6, dadurch gekennzeichnet, daß man die Hydrierung in Gegenwart eines 0,5 bis 2,0 Gew.-% Palladium und/oder Platin und gegebenenfalls 0,2 bis 0,8 Gew.-% Vanadin enthaltenden Katalysators durchführt.

8. Verfahren nach Ansprüchen 1 bis 7, dadurch gekennzeichnet, daß man die Hydrierung in Gegenwart eines Trägerkatalysators durchführt, bei dem der Träger ein Lithium-Aluminium-Spinell mit einer spezifischen Oberfläche von 20 bis 400 m²/g ist.

9. Verfahren nach Ansprüchen 1 bis 8, dadurch gekennzeichnet, daß man in die Hydrierung Acetonitril einsetzt, das als Nebenprodukt bei der Acrylnitril-Herstellung anfällt.

## Revendications

1. Procédé pour la préparation continue de triéthylamine par hydrogénation catalytique d'acétonitrile en phase gazeuse à des températures de 80 à 115°C et sous des pressions de 1 à 60 bars, ledit procédé étant caractérisé en ce que l'on effectue l'hydrogénation en présence d'un métal noble du groupe VIII de la classification périodique des éléments (Mendéléieff) sur une spinelle de lithium-aluminium comme support, éventuellement en présence de mono- et diéthylamine jusqu'à environ 5% en poids par rapport à l'acétonitrile.

2. Procédé selon la revendication 1, caractérisé en ce que l'on sépare par distillation la mono- et la diéthylamine contenues dans le mélange de réaction et on les renvoie dans l'hydrogénation.

3. Procédé selon les revendications 1 et 2, caractérisé en ce que l'on sépare par distillation la mono- et la diéthylamine, contenues dans le mélange de réaction en quantité allant jusqu'à environ 5% en poids après le premier passage, et on les renvoie à l'hydrogénation, la séparation de la mono- et de la diéthylamine et leur recyclage étant ensuite effectués en continu.

4. Procédé selon les revendications 1 à 3, caractérisé en ce que l'on effectue l'hydrogénation sur un catalyseur sur support qui contient 0,1 à 5% en poids d'un métal noble du groupe VIII de la classification péroidique des éléments.

5. Procédé selon les revendications 1 à 4, caractérisé en ce que l'on effectue l'hydrogénation en présence d'un catalyseur contenant en outre du vanadium.

6. Procédé selon les revendications 1 à 5, caractérisé en ce que l'on effectue l'hydrogénation en présence d'un catalyseur qui contient en outre 0,1 à 2,0% en poids de vanadium.

7. Procédé selon les revendications 1 à 6, caractérisé en ce que l'on effectue l'hydrogénation en présence d'un catalyseur contenant 0,5 à 2,0% en poids de palladium et/ou de platine et éventuellement 0,2 à 0,8% en poids de vanadium.

8. Procédé selon les revendications 1 à 7, caractérisé en ce que l'on effectue l'hydrogénation en présence d'un catalyseur sur support, dont le support est une spinelle de lithium-aluminium ayant une surface spécifique de 20 à 400 m²/g.

9. Procédé selon les revendications 1 à 8, caractérisé en ce que l'on utilise dans l'hydrogénation de l'acétonitrile qui se forme comme produit secondaire dans la préparation de l'acrylonitrile.

## Claims

1. Process for the continuous preparation of triethylamine by catalytic hydrogenation of acetonitrile in the gas phase at temperatures of 80 to 115°C and under pressures of 1 to 60 bars, characterised in that the hydrogenation is carried out in the presence of a noble metal of group VIII of the periodic system (Mendeleev) on lithium aluminium spinel as a support, if appropriate in the presence of up to about 5% by weight of monoethylamine and diethylamine, relative to acetonitrile.

2. Process according to Claim 1, characterised in that the monoethylamine and diethylamine contained in the reaction mixture are separated off by distillation and recycled into the hydrogenation.

3. Process according to Claims 1 and 2, characterised in that the monoethylamine and diethylamine contained in the reaction mixture in an amount of up to about 5% by weight after the first pass are separated off by distillation and recycled into the hydrogenation, the separating off and recycling of monoethylamine and diethylamine then being carried out continuously.

4. Process according to Claims 1 to 3, characterised in that the hydrogenation is carried out on a supported catalyst which contains 0.1 to 5% by weight of a noble metal of group VIII of the periodic system.

5. Process according to Claims 1 to 4, characterised in that the hydrogenation is carried out in the presence of a catalyst which additionally contains vanadium.

6. Process according to Claims 1 to 5, characterised in that the hydrogenation is carried out in the presence of a catalyst which additionally contains 0.1 to 2.0% by weight of vanadium.

7. Process according to Claims 1 to 6, characterised in that the hydrogenation is carried out in the presence of a catalyst containing 0.5 to 2.0% by weight of palladium and/or platinum and optionally 0.2 to 0.8% by weight of vanadium.

8. Process according to Claims 1 to 7, characterised in that the hydrogenation is carried out in the presence of a supported catalyst in which the support is a lithium aluminium spinel with a specific surface area of 20 to 400 $m^2/g$.

9. Process according to Claims 1 to 8, characterised in that acetonitrile which is obtained as a byproduct in the preparation of acrylonitrile is employed in the hydrogenation.